# FASCICULE DE BREVET EUROPEEN

(11) **EP 2 852 369 B1**
(45) Date de publication et mention de la délivrance du brevet: **13.03.2019**
(21) Numéro de dépôt: 13727345.4
(22) Date de dépôt: 21.05.2013
(51) Int. Cl.: A61K 8/02, A61K 8/19, A61K 8/26, A61K 8/29, A61K 8/88, A61Q 19/08

(54) **UTILISATION D'UNE COMPOSITION CONTENANT UN POLYMÈRE ET DES CHARGES MINÉRALES POUR LUTTER CONTRE LE VIEILLISSEMENT CUTANÉ**
VERWENDUNG EINER ZUSAMMENSETZUNG MIT EINEM POLYMER UND MINERALISCHEN FÜLLSTOFFEN GEGEN HAUTALTERUNG
USE OF A COMPOSITION CONTAINING A POLYMER AND MINERAL FILLERS TO COMBAT SKIN AGEING

(30) Priorité: 22.05.2012 FR 1254662
(43) Date de publication de la demande: 01.04.2015
(73) Titulaire: Rhodia Poliamida E Especialidades Ltda, Sao Paulo - SP (BR)
(72) Inventeur: CANOVA, Thomas, Gonzaga, Sao Paulo (BR); GORESCU, Gabriel, Sao Paulo (BR); CORDEIRO BASTOS, Tarcis, Sao Paulo (BR)
(74) Mandataire: Casalonga
(86) Numéro de dépôt international: PCT/IB2013/000992
(87) Numéro de publication internationale: WO 2013/175289

(56) Documents cités:
- EP-A1- 2 402 387
- US-A- 4 999 243
- US-A1- 2007 141 095
- US-B1- 6 316 102
- DATABASE WPI Week 200768 Thomson Scientific, London, GB; AN 2007-724646 XP002692151, & KR 2007 0006549 A (SAMSINSA CO LTD) 11 janvier 2007 (2007-01-11)
- DATABASE WPI Week 200712 Thomson Scientific, London, GB; AN 2007-119332 XP002692152, & KR 2006 0081174 A (AMOREPACIFIC CORP) 12 juillet 2006 (2006-07-12)

## Description

La présente invention a pour objet l'utilisation d'une composition polymérique, contenant une matrice polymérique et des charges minérales, uniformément dispersées dans cette matrice polymérique, qui possèdent des propriétés d'absorption et/ou d'émission de radiations dans le domaine de l'infrarouge lointain, afin de prévenir ou de diminuer les signes du vieillissement cutané.

On sait que l'apparence de la peau humaine évolue au cours du temps, en raison de phénomènes de vieillissements en général naturels, et qui peuvent être accélérés par des facteurs extérieurs tels que la pollution, le mode de vie (par exemple l'alimentation, le stress, le tabagisme).

Ce vieillissement de la peau se traduit en particulier par l'apparition à sa surface de diverses marques telles que des rides plus ou moins profondes, des taches dites « taches de vieillesse ».

Ces marques de vieillissement cutanées sont de plus en plus considérées comme inesthétiques, en particulier celles situées sur les parties apparentes du corps telles que le visage, le cou, les mains.

Ainsi, de nombreux produits cosmétiques ont été développés pour prévenir l'apparition des signes du vieillissement cutané. Ces produits se présentent en général sous la forme de compositions telles que des crèmes, des fluides plus ou moins épais, des sérums, contenant un ou plusieurs principes actifs qui sont des composés chimiques ou naturels destinés à lutter contre les rides et/ou les tâches. Ces compositions doivent en général être appliquées sur les zones à traiter une à deux fois par jour, et présentent des degrés d'efficacité très variable.

La demande de brevet KR 2007 0006549 décrit une composition cosmétique émettrice de rayonnement infrarouge lointain destinée à être appliquée sur la peau, et qui comprend de 5 à 10 % en poids de germanium, de 20 à 30 % en poids de zéolite, et de 1 à 5 % en poids de dioxyde de titane.

Malgré les nombreux produits actuellement présents sur le marché, le consommateur est toujours à la recherche de solutions innovantes et efficaces, pouvant être utilisées à la place ou en complément des solutions déjà existantes. Il existe ainsi un besoin de proposer de nouvelles solutions, qui permettent de lutter de manière efficace contre le vieillissement de la peau.

Poursuivant ses recherches dans ce domaine, la Demanderesse a maintenant découvert une approche nouvelle et originale, qui permet de combattre efficacement les signes du vieillissement cutané, en particulier les rides et les tâches de vieillesse.

Cette approche repose sur l'utilisation d'une composition polymérique particulière, comprenant une matrice de polymère au sein de laquelle sont dispersées des charges minérales qui émettent et/ou absorbent des radiations infrarouges dans la plage de longueur d'onde située entre 2 µm et 20 µm.

En effet la Demanderesse a découvert, de manière totalement inattendue, qu'une telle composition polymérique, lorsqu'elle était mise en contact avec la peau, avait pour effet de diminuer les signes de vieillissement déjà présents sur la peau, et de prévenir ou retarder l'apparition de nouveaux signes.

Ainsi, la présente invention a pour objet l'utilisation d'une composition polymérique contenant une matrice polymérique et au moins deux charges minérales de types différents choisis parmi les oxydes, les sulfates, les carbonates, les phosphates et les silicates, uniformément dispersées dans la matrice polymérique, ayant des propriétés d'absorption et/ou d'émission dans la région d'infrarouge lointain allant de 2 µm à 20 µm, pour prévenir ou diminuer les rides et/ou les taches de vieillesse de la peau, caractérisée en ce que la composition polymérique se présente sous la forme de particules dispersées dans une composition cosmétique.

La présente invention a également pour objet une méthode pour prévenir ou diminuer les rides et/ou les taches de vieillesse de la peau, consistant à mettre la peau au contact d'une composition polymérique telle que décrite dans la présente demande.

L'invention met en oeuvre une composition polymérique comprenant une matrice polymérique.

La matrice polymérique peut être choisie en particulier dans le groupe comprenant : les polyesters, les polyoléfines, les polymères à base de cellulose-ester tels que l'acétate de cellulose, le propionate de cellulose, le rayon, la viscose et les polymères de la même famille, les polymères et copolymères acryliques, les polyamides tels que le polyhexaméthylène adipamide (PA66) le polycaproamide (PA6), les PA6.10, PA10.10 et PA12, les copolymères en toutes proportions de ces polymères, et les mélanges entre n'importe lesquels de ces polymères.

Selon une forme préférentielle de réalisation, la matrice polymérique est constituée de polyamide, de préférence choisi entre le polyamide 6, le polyamide 66 et les copolymères de polyamide 6/polyamide 66 en toutes proportions.

La composition selon l'invention comprend au moins deux charges minérales ayant des propriétés d'absorption et/ou d'émission dans la région d'infrarouge lointain allant de 2 à 20 µm. De préférence, les charges minérales ont des propriétés d'absorption et/ou d'émission dans la région d'infrarouge lointain allant de 3 à 20 µm, et encore plus préférentiellement de 3 à 15 µm.

Selon l'invention, les charges minérales sont dispersées uniformément dans la matrice polymérique. Par « uniformément dispersées » on entend que les charges minérales sont incorporées de manière homogène au sein même du polymère. En particulier, les particules sont piégées dans la composition de polymère. Il ne s'agit donc pas de charges minérales déposées sur le polymère, par exemple sous la forme d'un revêtement à la surface du polymère (« coating »).

Une telle dispersion uniforme peut être obtenue en incorporant les charges minérales dans le polymère lors de la synthèse de ce dernier. Un mode de réalisation consiste à réaliser une ou plusieurs suspensions de charges minérales, stabilisée(s) par des tensioactifs. La (les) suspension(s) est(sont) ensuite ajoutée(s) au cours de la synthèse du polymère.

L'on peut également incorporer lesdites charges par mélange de celles-ci au polymère fondu, soit directement, soit au moyen d'un concentré de particules sous forme de mélange maître (« masterbatch »), celui-ci pouvant être postérieurement dilué en concentrations prédéterminées dans la masse polymérique. Cette incorporation dans le polymère fondu peut être avantageusement effectuée au moment de la mise en forme de la composition polymérique, par exemple au moment de l'extrusion de la composition polymérique.

Grâce à de tels procédés l'on peut obtenir des compositions de polymère selon l'invention qui contiennent les charges minérales de façon uniformément dispersée dans la matrice polymère.

Les charges minérales utilisables selon l'invention sont choisies parmi les oxydes, les sulfates, les carbonates, les phosphates et les silicates.

De préférence, le ou les oxyde(s) est (sont) choisi(s) parmi le dioxyde de titane, le dioxyde de silicium et l'oxyde de magnésium.

Le ou les sulfate(s) peuvent être avantageusement choisi(s) parmi les sulfates de métaux alcalins et de métaux alcalino-terreux, de préférence parmi le sulfate de baryum, le sulfate de calcium et le sulfate de strontium.

Le ou les carbonate(s) est (sont) choisi(s) avantageusement parmi le carbonate de calcium ou de sodium.

De préférence, le ou les silicate(s) est(sont) choisi(s) parmi l'actinolite, la tourmaline, la serpentine, le kaolinite, et le silicate de zirconium.

Le ou les phosphate(s) peuvent être choisi(s) parmi les phosphates de zirconium, le phosphate de cérium, l'apatite et leurs mélanges.

La composition polymérique contient au moins deux charges minérales de types différents, choisis parmi les types suivants : les oxydes, les sulfates, les carbonates, les phosphates et les silicates. De manière particulièrement préférée, la composition polymérique contient au moins trois charges minérales de types différents, choisis parmi les types énoncés ci-avant.

Selon un premier mode de réalisation préféré, la composition polymérique contient au moins deux charges minérales de types différents, choisis parmi les types suivants : les oxydes, les sulfates, et les silicates, et de préférence parmi le dioxyde de titane, un sulfate de métal alcalin ou alcalino-terreux et un silicate, et de manière encore plus préférée parmi le dioxyde de titane, le sulfate de baryum, et la tourmaline.

De manière plus préférée, la composition polymérique contient au moins trois charges minérales de types différents choisis parmi les types ci-avant. De manière particulièrement préférée, la composition polymérique contient trois charges minérales de types différents, qui sont un oxyde, un sulfate, et un silicate.

On préfère tout particulièrement l'association dioxyde de titane/sulfate de métal alcalino-terreux/silicate; et encore plus préférentiellement l'association dioxyde de titane/ sulfate de baryum/ tourmaline.

Dans ce cas, les proportions respectives en poids des trois charges minérales ci-avant sont de préférence comprises entre 80:10:10 et 10:30:60, et plus spécifiquement ces proportions respectives sont de 50:25:25.

Selon un second mode de réalisation, également avantageux, la composition polymérique contient au moins deux charges minérales de types différents, et de préférence au moins trois charges minérales de types différents choisies parmi les types suivants : les oxydes, les phosphates, et les silicates.

Dans ce mode de réalisation, on préfère en particulier les associations de trois charges minérales de types différents, à savoir un oxyde, un phosphate, et un silicate.

De préférence, la proportion en poids de charge(s) minérale(s) par rapport au poids total de la composition polymérique est supérieure ou égale à 1,0 %, de préférence supérieure ou égale à 1,5 % et plus préférentiellement encore supérieure ou égale a 2,5 %.

De préférence, la proportion en poids de charge(s) minérale(s) par rapport au poids total de la composition polymérique est inférieure ou égale à 50 %, de préférence inférieure ou égale à 40 %, et plus préférentiellement encore inférieure ou égale à 30 %.

La ou les charges minérales selon l'invention se présentent avantageusement sous forme de particules, lesquelles présentent de préférence une taille moyenne en volume inférieure ou égale à 2 µm, mesurée selon la méthode d'analyse granulométrique par diffraction laser (en utilisant, par exemple, des granulomètres MALVERN ou CILAS).

Une manière avantageuse de procéder consiste à mettre les particules en suspension dans l'eau, et à déterminer leur granulométrie par diffraction laser en employant la méthode décrite dans la norme ISO 13320 :2009.

Il est préférable que les charges minérales utilisées dans la présente invention présentent une taille de particules qui ne soit :
- ni trop petite, pour prévenir tout risque que les particules ne puissent sortir de la matrice polymérique et s'introduire dans le corps humain au travers de la peau ou via les voies respiratoires, ou encore se disperser dans l'environnement ;
- ni trop grosse, ce qui rendrait plus difficile l'incorporation des particules dans la matrice polymérique et surtout pourrait rendre la composition abrasive au contact de la peau, ce qui peut être inconfortable pour l'utilisateur, voire dans certains cas pourrait risquer d'avoir un effet irritant sur la peau, par exemple dans le cas de peaux particulièrement fines ou sensibles.

Ainsi, la ou les charges minérales selon l'invention se présentent sous forme de particules qui présentent avantageusement une taille moyenne en volume, mesurée selon la méthode d'analyse granulométrique par diffraction laser, allant de 0,1 à 2 µm, plus préférentiellement de 0,2 à 1,5 µm, et encore plus préférentiellement de 0,2 à 1 µm.

Les charges minérales présentent avantageusement une distribution granulométrique avec 99 % en volume des particules ayant une taille inférieure à 1,0 µm, de préférence 90 % en volume des particules ayant une taille inférieure à 0,5 µm. La distribution granulométrique est également mesurée par la méthode d'analyse granulométrique par diffraction laser précitée (en utilisant, par exemple, des granulomètres MALVERN ou CILAS).

La composition polymérique selon l'invention présente de préférence un nombre de pics d'absorption de radiations infrarouges supérieur à 10 dans les dix plages de fréquence suivantes : 3,00 +/-0,30µm, 6,20 +/- 0,50µm, 8,00 +/- 0,25µm, 8,50 +/- 0,25µm, 9,00 +/-0,25µm, 9,50 +/- 0,25µm, 10,00 +/- 0,25µm, 10,50 +/- 0,25µm, 11,00 +/- 0,25µm, 14,60 +/- 2,10µm, au moins 1 pic étant présent dans au moins 7 de ces dix plages de fréquence.

Le spectre d'absorption de radiations infrarouges peut être déterminé par toute méthode connue de l'homme du métier. Une méthode possible est l'utilisation d'un appareil Bruker Equinox 55, avec une résolution de 4 cm⁻¹. Dans ce cas le spectre obtenu est sous forme ATR («Atenuated Total Reflectance»), en utilisant un cristal ZnSe.

La composition polymérique se présente sous la forme de particules dispersées dans une composition cosmétique telle qu'une crème, un fluide, un sérum, une composition de maquillage qui peut être solide (poudre, rouge à lèvre) ou fluide.

Les particules de composition polymérique présentent avantageusement une taille moyenne en volume inférieure ou égale à 250 µm, de préférence allant de 5 à 150 µm, et plus préférentiellement de 10 à 50 µm.

La taille moyenne en volume des particules de composition polymérique est mesurée selon la méthode d'analyse granulométrique par diffraction laser décrite ci-avant (en utilisant, par exemple, des granulomètres MALVERN ou CILAS).

Le ratio entre la taille moyenne en volume des particules de composition polymérique et la taille moyenne en volume des charges minérales peut être optimisé pour éviter tout risque que les particules, trop petites, ne puissent sortir de la matrice polymérique et s'introduire dans le corps humain ou se disperser dans l'environnement, ou au contraire ne soient trop grosses, avec le risque de rendre la composition abrasive au contact de la peau.

Ainsi, le ratio entre la taille moyenne en volume des particules de composition polymérique selon l'invention et la taille moyenne en volume des charges minérales, ces deux tailles étant mesurées selon la méthode d'analyse granulométrique par diffraction laser, est alors avantageusement supérieur ou égal à 5. Ce ratio est de préférence inférieur ou égale à 250. Ce ratio va de préférence de 5 à 150, et plus préférentiellement de 5 à 100.

Les particules de composition polymérique selon l'invention peuvent être préparées par les méthodes connues de l'homme du métier pour l'obtention de poudres ou fines particules de polymères, par exemple par broyage, cryo-broyage ou séchage par pulvérisation (« spay-drying ») de la composition polymérique. L'on peut employer de manière alternative la méthode décrite dans la demande de brevet FR 2 899 591 au nom de la Demanderesse.

Comme expliqué ci-avant, les particules de composition polymérique selon l'invention sont utilisées sous forme d'une dispersion au sein d'une composition cosmétique.

Cette dernière peut comprendre en particulier un solvant qui peut être choisi parmi l'eau, les fluides organiques, et les mélanges d'eau et de fluides organiques miscibles ou non miscibles à l'eau.

La composition cosmétique peut également comprendre tous les ingrédients classiques, connus de l'homme du métier comme entrant dans la composition de crèmes ou fluides cométiques pour la peau, tels que par exemple et de manière non limitative des agents épaississants, des agents hydratants, des filtres UV, des agents antioxydants.

Selon un mode de réalisation particulièrement avantageux, ladite composition cosmétique comprend également un ou plusieurs principes actifs anti-rides ou anti-tâches, différents des charges minérales selon l'invention.

En effet, dans ce cas, il a également été constaté un effet de synergie entre les particules de composition polymérique selon l'invention et les principes actifs anti-rides ou anti-tâches.

La composition cosmétique contenant les particules de composition polymérique selon l'invention est utilisée en appliquant ladite composition sur la peau, sur la ou les zones à traiter. Cette application peut être quotidienne, bi-quotidienne (par exemple, le matin et le soir), ou plus épisodique (tous les deux jours, une fois par semaine, ....).

Après application sur la peau, la composition peut être soit laissée, soit rincée après un temps de pose qui peut aller de quelques minutes à quelques heures.

La présente invention a également pour objet une méthode de traitement cosmétique de la peau, pour prévenir ou diminuer les rides et/ou les taches de vieillesse de la peau, consistant à mettre la peau en contact avec une composition polymérique telle que décrite dans la présente demande.

La description détaillée faite ci-avant de l'utilisation selon l'invention s'applique également à la méthode selon l'invention.

On donne ci-après un exemple de réalisation de l'invention, à titre illustratif.

### EXEMPLE

Dans cet exemple, les matières premières utilisées sont les suivantes :
- Polymère PA66 de viscosité relative 2,6 ;
- Tourmaline (taille moyenne en volume de particule de 0,8 µm) ;
- Sulfate de baryum (taille moyenne en volume de particule de 0,8 µm) ;
- Dioxyde de titane (taille moyenne en volume de particule de 0,3 µm) ;
- Additif A : Copolymère polyamide/polyoxyde d'alkylène étoile hydrophile obtenu de la façon suivante :
   Dans une autoclave de 7,5 litres équipée d'un agitateur mécanique sont introduits: 1116,0 g d'a-caprolactame (9,86 mol), 57,6 g d'acide 1,3,5-benzène tricarboxylique (0,27mol), 1826,4 g de Jeffamine M2070 (0,82 mol), 1,9g d'ULTRANOX 236 et 3,5 g d'une solution aqueuse à 50% (p/p) d'acide hypophosphoreux.

Le mélange réactionnel est porté à 250°C sous azote et sous pression atmosphérique et maintenu à cette température pendant 1h. Puis le système est progressivement mis sous vide pendant 30 min jusqu'à une pression de 5 mbars, puis maintenu sous vide pendant une heure supplémentaire. Le systéme est ensuite coulé sur un plateau.
- Polyoxyde d'éthylène de poids moléculaire 400g/mole.

### Préparation de la composition de polymère :

Le polyamide est mélangé à la tourmaline, au sulfate de baryum et au dioxyde de titane de façon à ce que la composition massique finale soit de 70% de PA66, 2,7% de tourmaline, 6,8% de sulfate de baryum et 20,5% de dioxyde de titane. Le mélange est refondu dans une extrudeuse double vis à température de 290°C et extrudé pour obtenir le polymère granulé.

### Préparation de particules de composition polymérique de taille 10 um :

On introduit dans une extrudeuse double vis 24D de type PRISM des granulés de la composition de polymère obtenue ci-avant à l'aide d'une alimentation volumétrique ainsi qu'un mélange de pastilles de l'additif A (concentration massique de 5%) et de polyoxyde d'éthylène (concentration massique de 19%) l'aide d'une alimentation pondérale. Le mélange est extrudé à un débit fixé entre 2,0 kg /heure. Les températures des différentes plages de l'extrudeuse sont comprises entre 275 et 295°C. La vitesse est fixée à 200 rpm. La pression enregistrée est comprise entre 10 et 13 bars. Les joncs obtenus sont trempés en sortie de filière par un flux d'eau, recueillis dans une panière métallique, égouttés puis séchés.

Les joncs collectés sont ensuite dispersés dans l'eau par simple agitation mécanique. La dispersion ainsi obtenue est tamisée avec un tamis 200 µm pour éliminer les impuretés solides de grande taille telles que des morceaux de jonc non-dispersables. Les rendements pondéraux de récupération de polymère polyamide après tamisage sont supérieurs à 90%. La distribution granulométrique des particules contenues dans la dispersion a été mesurée à l'aide d'un appareil dénommé MasterSize 2000 commercialisé par la société Malvern Instruments. Cette distribution, exprimée en volume, obtenue après application d'Ultrasons, est unimodale et la valeur du pic modal est 10 µm.

L'on obtient ainsi des particules de composition polymérique contenant 70% en poids de PA66 et 30% en poids de charges minérales (dioxyde de titane, sulfate de baryum, tourmaline).

### Préparation d'une composition cosmétique anti-rides contenant les particules de composition polymérique:

On a préparé une composition anti-rides pour le visage, à partir des ingrédients indiqués dans le tableau ci-dessous (la teneur de chaque ingrédient étant indiquée en pourcentage en poids, par rapport au poids total de la composition).

Les particules de composition polymérique employées sont celles préparées selon le descriptif ci-avant.

| Ingrédients | Teneur en poids |
|---|---|
| *Phase A* | |
| Cyclopentasiloxane/ PEG/PPG-20/15 Dimethicone (produit commercialisé sous la dénomination SF1528 par la société Kobo Products) | 11,00% |
| Cyclopentasiloxane (produit commercialisé sous la dénomination SF1202 par la sociéeté Kobo Products) | 9,00% |
| Cyclopentasiloxane/Diméthicone (produit commercialisé sous la denomination SF1214 par la societé Kobo) | 7,50% |

| *Phase B* | |
|---|---|
| Particules de polyamide 66 contenant des charges minérales | 7,50% |

| *Phase C* | |
|---|---|
| Glycérine (Glycerin U.S.P. Natural 96%, commercialisé par la société Univar USA Inc.) | 8,00% |
| Chlorure de sodium | 1,00% |
| Butylène glycol/Eau/Palmitoyl hydroxypropyltrimonium, amylopectine/Polymère de glycérine /Polysorbate20/ Rétinol/ Phénoxyéthanol/Parabènes/ Lécithine hydrogénée/BHT/BHA (produit commercialisé sous la dénomination Gs-VA100C par la société Kobo Products) | 0,50% |
| Eau/ papaine /Palmitoyl hydroxypropyltrimonium amylopectine/Polymère réticulé de glycérine / Phénoyéthanol/Lécithine hydrogénée /Parabènes (produit commercialisé sous la dénomination GsPPY par la société Kobo Products) | 0,50% |
| Polysorbate 80 (produit commercialisé sous la dénomination Liposorb O-20 par la société LIPO Chemicals) | 0,20% |
| Quaternium-15 (produit commercialisé sous la dénomination Dowicil 200 par la societé DOW Chemical) | 0,10% |
| Eau déionisée | qsp 100% |

Cette composition a été préparée de la manière suivante : les composés de la phase A ont été mélangés, et le mélange homogénéisé pendant 15 minutes. Puis les particules de polymère (phase B) ont été ajoutées, et l'homogénéisation poursuivie pendant 15 minutes.

Les ingrédients de la phase C ont été pré-mélangés à part, puis ajoutées progressivement au mélange principal en cinq portions, en respectant un temps de mélange de 15-20 minutes entre chaque ajout.

Après homogénéisation complète du mélange, la composition ainsi obtenue a alors été conditionnée, en la versant dans des récipients appropriés.

## Revendications

1. Utilisation d'une composition polymérique contenant une matrice polymérique et au moins deux charges minérales de types différents choisis parmi les oxydes, les sulfates, les carbonates, les phosphates et les silicates, uniformément dispersées dans la matrice polymérique, ayant des propriétés d'absorption et/ou d'émission dans la région d'infrarouge lointain allant de 2 µm à 20 µm, pour prévenir ou diminuer les rides et/ou les taches de vieillesse de la peau, **caractérisée en ce que** la composition polymérique se présente sous la forme de particules dispersées dans une composition cosmétique.

2. Utilisation selon la revendication précédente, **caractérisée en ce que** la matrice polymérique est choisie dans le groupe comprenant les polyesters, les polyoléfines, les polymères à base de cellulose-ester, les polymères et copolymères acryliques, les polyamides, leurs copolymères et leurs mélanges.

3. Utilisation selon la revendication précédente, **caractérisée en ce que** la matrice polymérique est constituée de polyamide, de préférence choisi entre le polyamide 6, le polyamide 66 et les copolymères de polyamide 6/polyamide 66 en toutes proportions.

4. Utilisation selon l'une quelconque des revendications précédentes, **caractérisée en ce que** la composition polymérique contient au moins trois charges minérales de types différents, choisis parmi les types suivants : les oxydes, les sulfates, les carbonates, les phosphates et les silicates.

5. Utilisation selon l'une quelconque des revendications précédentes, **caractérisée en ce que** la composition polymérique contient au moins deux charges minérales de types différents, et de préférence au moins trois charges minérales de types différents, choisis parmi les types suivants : les oxydes, les sulfates, et les silicates, et de préférence parmi le dioxyde de titane, un sulfate de métal alcalin ou alcalino-terreux et un silicate, et de manière encore plus préférée parmi le dioxyde de titane, le sulfate de baryum, et la tourmaline.

6. Utilisation selon la revendication précédente, **caractérisée en ce que** la composition polymérique contient trois charges minérales de types différents, qui sont un oxyde, un sulfate, et un silicate, et plus préférentiellement l'association dioxyde de titane/sulfate de baryum/tourmaline.

7. Utilisation selon la revendication 1, **caractérisée en ce que** la composition polymérique contient au moins deux charges minérales de types différents, et de préférence au moins trois charges minérales de types différents choisies parmi les types suivants : les oxydes, les phosphates, et les silicates.

8. Utilisation selon la revendication précédente, **caractérisée en ce que** la composition polymérique contient trois charges minérales de types différents, qui sont un oxyde, un phosphate, et un silicate.

9. Utilisation selon l'une quelconque des revendications précédentes, **caractérisée en ce que** la proportion en poids de charge(s) minérale(s) par rapport au poids total de la composition polymérique est supérieure ou égale à 1,0 %, de préférence supérieure ou égale à 1,5 % et plus préférentiellement encore supérieure ou égale a 2,5 %.

10. Utilisation selon l'une quelconque des revendications précédentes, **caractérisée en ce que** la proportion en poids de charge(s) minérale(s) par rapport au poids total de la composition polymérique est inférieure ou égale à 50 %, de préférence inférieure ou égale à 40%, et plus préférentiellement encore inférieure ou égale à 30 %.

11. Utilisation selon l'une quelconque des revendications précédentes, **caractérisée en ce que** la ou les charges minérales se trouvent sous forme de particules présentant une taille moyenne en volume, mesurée selon la méthode d'analyse granulométrique par diffraction laser, inférieure ou égale à 2 µm, de préférence allant de 0,1 à 2 µm, plus préférentiellement de 0,2 à 1,5 µm, et encore plus préférentiellement de 0,2 à 1 µm.

12. Utilisation selon l'une des revendications 1 à 11, **caractérisée en ce que** la composition cosmétique est une crème, un fluide, un sérum, une composition de maquillage solide ou fluide.

13. Utilisation selon la revendication 1, **caractérisée en ce que** les particules de composition polymérique présentent une taille moyenne en volume, mesurée selon la méthode d'analyse granulométrique par diffraction laser, inférieure ou égale à 250 µm, de préférence allant de 5 à 150 µm, et plus préférentiellement de 10 à 50 µm.

14. Méthode pour prévenir ou diminuer les rides et/ou les taches de vieillesse de la peau, consistant à mettre la peau au contact d'une composition polymérique telle que définie dans l'une quelconque des revendications précédentes.

## Patentansprüche

1. Verwendung einer Polymerzusammensetzung, die eine Polymermatrix und mindestens zwei mineralische Füllstoffe verschiedener Art, die aus Oxiden, Sulfaten, Carbonaten, Phosphaten und Silikaten ausgewählt sind, die einheitlich in der Polymermatrix dispergiert sind und Absorptions- und/oder Emissionseigenschaften im Ferninfrarotbereich im Bereich von 2 *µ*m bis 20 *µ*m aufweisen, umfasst zur Prävention oder Verringerung von Falten und/oder Altersflecken der Haut, **dadurch gekennzeichnet, dass** die Polymerzusammensetzung in Form von in einer kosmetischen Zusammensetzung dispergierten Teilchen vorliegt.

2. Verwendung nach dem vorhergehenden Anspruch, **dadurch gekennzeichnet, dass** die Polymermatrix aus der Gruppe umfassend Polyester, Polyolefine, Polymere auf Celluloseesterbasis, Acrylpolymere und -copolymere, Polyamide, Copolymere davon und Mischungen davon ausgewählt ist.

3. Verwendung nach dem vorhergehenden Anspruch, **dadurch gekennzeichnet, dass** die Polymermatrix aus Polyamid, das vorzugsweise aus Polyamid 6, Polyamid 66, und Copolymeren von Polyamid 6 und Polyamid 66 in allen Verhältnissen ausgewählt ist, besteht.

4. Verwendung nach einem der vorhergehenden Ansprüche, **dadurch gekennzeichnet, dass** die Polymerzusammensetzung mindestens drei mineralische Füllstoffe verschiedener Art enthält, die aus den folgenden Arten ausgewählt sind: Oxide, Sulfate, Carbonate, Phosphate und Silikate.

5. Verwendung nach einem der vorhergehenden Ansprüche, **dadurch gekennzeichnet, dass** die Polymerzusammensetzung mindestens zwei mineralische Füllstoffe verschiedener Art und vorzugsweise mindestens drei mineralische Füllstoffe verschiedener Art enthält, die aus den folgenden Arten ausgewählt sind: Oxide, Sulfate und Silikate, und vorzugsweise aus Titandioxid, einem Alkalimetall- oder Erdalkalimetallsulfat und einem Silikat und noch weiter bevorzugt aus Titandioxid, Bariumsulfat und Turmalin.

6. Verwendung nach dem vorhergehenden Anspruch, **dadurch gekennzeichnet, dass** die Polymerzusammensetzung drei mineralische Füllstoffe verschiedener Art enthält, bei denen es sich um ein Oxid, ein Sulfat und ein Silikat und weiter bevorzugt um die Kombination Titandioxid/Bariumsulfat/Turmalin handelt.

7. Verwendung nach Anspruch 1, **dadurch gekennzeichnet, dass** die Polymerzusammensetzung mindestens zwei mineralische Füllstoffe verschiedener Art und vorzugsweise mindestens drei mineralische Füllstoffe verschiedener Art enthält, die aus den folgenden Arten ausgewählt sind: Oxide, Phosphate und Silikate.

8. Verwendung nach dem vorhergehenden Anspruch, **dadurch gekennzeichnet, dass** die Polymerzusammensetzung drei mineralische Füllstoffe verschiedener Art enthält, bei denen es sich um ein Oxid, ein Phosphat und ein Silikat handelt.

9. Verwendung nach einem der vorhergehenden Ansprüche, **dadurch gekennzeichnet, dass** der Gewichtsanteil von mineralischem Füllstoff bzw. mineralischen Füllstoffen am Gesamtgewicht der Polymerzusammensetzung größer oder gleich 1,0 %, vorzugsweise größer oder gleich 1,5 % und noch weiter bevorzugt größer oder gleich 2,5 % ist.

10. Verwendung nach einem der vorhergehenden Ansprüche, **dadurch gekennzeichnet, dass** der Gewichtsanteil von mineralischem Füllstoff bzw. mineralischen Füllstoffen am Gesamtgewicht der Polymerzusammensetzung kleiner oder gleich 50 %, vorzugsweise kleiner oder gleich 40 % und noch weiter bevorzugt kleiner oder gleich 30 % ist.

11. Verwendung nach einem der vorhergehenden Ansprüche, **dadurch gekennzeichnet, dass** der mineralische Füllstoff bzw. die mineralischen Füllstoffe in Form von Teilchen mit einer gemäß der Methode der Teilchengrößenanalyse durch Laserbeugung gemessenen volumenmittleren Größe kleiner oder gleich 2 *µ*m, vorzugsweise im Bereich von 0,1 bis 2 *µ*m, weiter bevorzugt von 0,2 bis 1,5 *µ*m und noch weiter bevorzugt von 0,2 bis 1 *µ*m vorliegt bzw. vorliegen.

12. Verwendung nach einem der Ansprüche 1 bis 11, **dadurch gekennzeichnet, dass** es sich bei der kosmetischen Zusammensetzung um eine Creme, ein Fluid, ein Serum oder eine feste oder flüssige Makeup-Zusammensetzung handelt.

13. Verwendung nach Anspruch 1, **dadurch gekennzeichnet, dass** die Polymerteilchen eine gemäß der Methode der Teilchengrößenanalyse durch Laserbeugung gemessene volumenmittlere Größe kleiner oder gleich 250 *µ*m, vorzugweise im Bereich von 5 bis 150 *µ*m und weiter bevorzugt von 10 bis 50 *µ*m aufweisen.

14. Verfahren zur Prävention oder Verringerung von Falten und/oder Altersflecken der Haut, das darin besteht, dass man die Haut mit einer Polymerzusammensetzung gemäß einem der vorhergehenden Ansprüche in Kontakt bringt.

## Claims

1. Use of a polymeric composition containing a polymer matrix and at least two mineral fillers of different types chosen from oxides, sulfates, carbonates, phosphates and silicates, uniformly dispersed in the polymer matrix, having properties of absorption and/or emission in the far infrared region ranging from 2 *µ*m to 20 *µ*m, for preventing or reducing wrinkles and/or age spots of the skin, **characterized in that** the polymeric composition is in the form of particles dispersed in a cosmetic composition.

2. Use according to the preceding claim, **characterized in that** the polymer matrix is chosen from the group comprising polyesters, polyolefins, polymers based on cellulose esters, acrylic polymers and copolymers, polyamides, copolymers thereof and blends thereof.

3. Use according to the preceding claim, **characterized in that** the polymer matrix consists of polyamide, preferably chosen from polyamide 6, polyamide 66 and copolymers of polyamide 6/polyamide 66 in any proportions.

4. Use according to any one of the preceding claims, **characterized in that** the polymeric composition contains at least three mineral fillers of different types, chosen from the following types: oxides, sulfates, carbonates, phosphates and silicates.

5. Use according to any one of the preceding claims, **characterized in that** the polymeric composition contains at least two mineral fillers of different types, and preferably at least three mineral fillers of different types, chosen from the following types: oxides, sulfates and silicates, and preferably from titanium dioxide, an alkali metal or alkaline-earth metal sulfate and a silicate, and even more preferably from titanium dioxide, barium sulfate and tourmaline.

6. Use according to the preceding claim, **characterized in that** the polymeric composition contains three mineral fillers of different types, which are an oxide, a sulfate and a silicate, and more preferentially the titanium dioxide/barium sulfate/tourmaline combination.

7. Use according to Claim 1, **characterized in that** the polymeric composition contains at least two mineral fillers of different types, and preferably at least three mineral fillers of different types, chosen from the following types: oxides, phosphates and silicates.

8. Use according to the preceding claim, **characterized in that** the polymeric composition contains three mineral fillers of different types, which are an oxide, a phosphate and a silicate.

9. Use according to any one of the preceding claims, **characterized in that** the weight proportion of mineral filler (s) relative to the total weight of the polymeric composition is greater than or equal to 1.0%, preferably greater than or equal to 1.5% and even more preferentially greater than or equal to 2.5%.

10. Use according to any one of the preceding claims, **characterized in that** the weight proportion of mineral filler(s) relative to the total weight of the polymeric composition is less than or equal to 50%, preferably less than or equal to 40% and even more preferentially less than or equal to 30%.

11. Use according to any one of the preceding claims, **characterized in that** the mineral filler(s) is (are) in the form of particles which have a volume-average size, measured according to the laser diffraction particle size analysis method, of less than or equal to 2 *µ*m, preferably ranging from 0.1 to 2 *µ*m, more preferentially from 0.2 to 1.5 *µ*m and even more preferentially from 0.2 to 1 *µ*m.

12. Use according to one of Claims 1 to 11, **characterized in that** the cosmetic composition is a cream, a fluid, a serum, or a solid or fluid makeup composition.

13. Use according to Claim 1, **characterized in that** the particles of polymeric composition have a volume-average size, measured according to the laser diffraction particle size analysis method, of less than or equal to 250 *µ*m, preferably ranging from 5 to 150 *µ*m and more preferentially from 10 to 50 *µ*m.

14. Method for preventing or reducing wrinkles and/or age spots of the skin, consisting in bringing the skin into contact with a polymeric composition as defined in any one of the preceding claims.
